# FASCICULE DE BREVET EUROPEEN

(11) **EP 2 544 620 B1**
(45) Date de publication et mention de la délivrance du brevet: **09.09.2015**
(21) Numéro de dépôt: 11712958.5
(22) Date de dépôt: 03.03.2011
(51) Int. Cl.: A61C 1/14, A61C 3/03, A61C 17/20, A61B 17/32, A61C 1/10

(54) **INSTRUMENT VIBRATOIRE A OUTIL INTERCHANGEABLE**
SCHWINGUNGSINSTRUMENT MIT AUSWECHSELBAREM WERKZEUG
VIBRATORY INSTRUMENT INCLUDING AN INTERCHANGEABLE TOOL

(30) Priorité: 12.03.2010 FR 1051803
(43) Date de publication de la demande: 16.01.2013
(73) Titulaire: SOCIETE POUR LA CONCEPTION DES APPLICATIONS DES TECHNIQUES ELECTRONIQUES, 33700 Merignac (FR); Lesage, Patrick, 35400 Saint Malo (FR)
(72) Inventeur: LESAGE, Patrick, 35400 Saint Malo (FR); RICHER, Jean-Michel, 33520 Bruges (FR); PINEL, Alain, 33127 Martignas Sur Jalle (FR)
(74) Mandataire: Desormiere, Pierre-Louis
(86) Numéro de dépôt international: PCT/FR2011/050444
(87) Numéro de publication internationale: WO 2011/110774

(56) Documents cités:
- EP-A1- 2 022 437
- DE-A1- 19 947 325
- US-A- 4 984 985

## Description

### Domaine et arrière plan de l'invention

La présente invention concerne les appareils de traitement dentaire et plus particulièrement les appareils à ultrasons, tels que les appareils de détartrage, de surfaçage (élimination de biofilms) ou de taille (cavités ou préparations de prothèse), etc., qui comprennent des instruments vibrant à des fréquences ultrasonores.

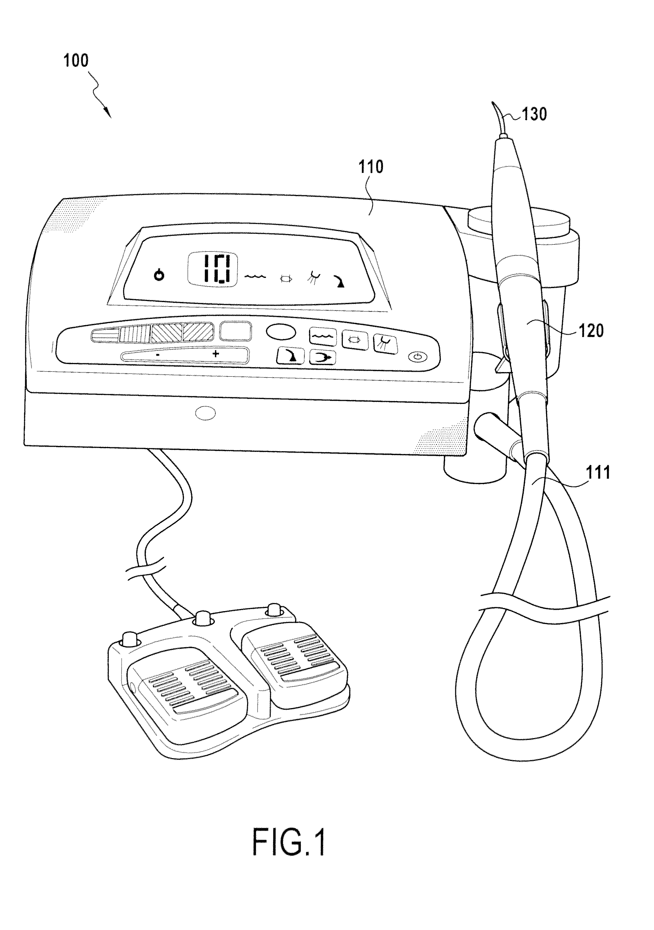

La figure 1 illustre un appareil de traitement à ultrasons 100 qui comprend un générateur d'ultrasons 110 relié à une pièce à main 120 par un cordon 111. Un instrument vibratoire 130, appelé "sonotrode" ou "insert" ("tip" en anglais) et destiné à vibrer à des fréquences sonores ou ultrasonores, est monté sur la partie supérieure de la pièce à main 120. De façon bien connue, la pièce à main 120 comprend un transducteur (non représenté) formé par exemple d'un matériau piézoélectrique et couplé mécaniquement à l'insert 130 de manière à transmettre à ce dernier des ondes vibratoires dont l'amplitude est déterminée en fonction de la puissance fournie par le générateur d'ultrasons 110.

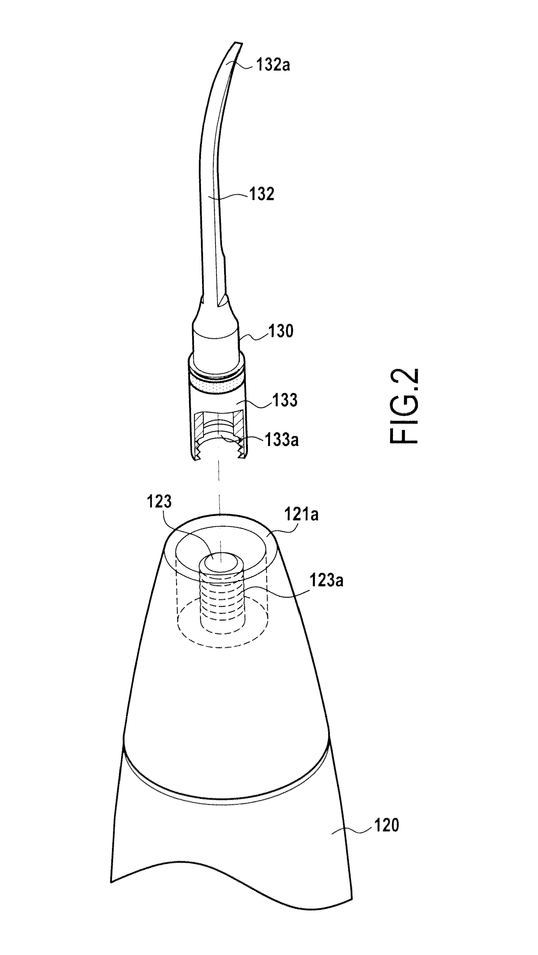

Comme illustré sur la figure 2, l'instrument vibratoire 130 est principalement formé de deux parties, une base ou partie proximale 133 qui est destinée à être fixée de façon rigide sur un élément 123 solidaire du transducteur (non représenté) de la pièce à main 120 et une partie travaillante ou outil 132 qui est destiné à reproduire les vibrations transmises par la pièce à main 120. La fixation de l'instrument vibratoire 130 sur la pièce à main 120 est généralement réalisée par vissage en serrant la base 133 de l'instrument qui comprend un taraudage 133a sur un élément de fixation 123 solidaire du transducteur et comprenant un filetage correspondant 123a.

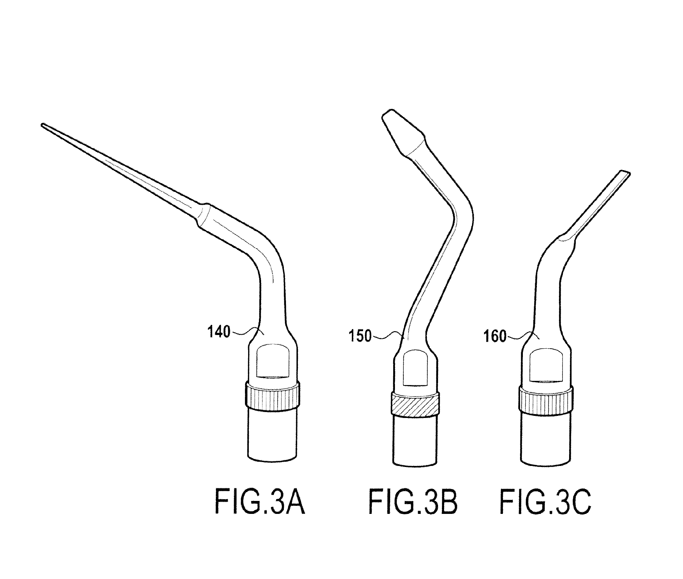

La partie travaillante ou outil 132 correspond à la partie "utile" de l'instrument, c'est-à-dire à la partie avec laquelle le traitement est réalisé. La forme de la partie travaillante de l'instrument et en particulier celle de son extrémité 132a est déterminée en fonction du traitement à réaliser. A titre d'exemple, l'instrument 130 présente une partie travaillante 132 dont la forme de son extrémité 132a est adaptée pour un traitement de détartrage. Les figures 3A à 3C illustrent des exemples d'instruments dont les parties travaillantes ou outils ont des formes adaptées pour réaliser respectivement des traitements de détartrage/débridement (insert 140, figure 3A), des traitements de préparation avant pose de prothèse (insert 150, figure 3B) et des traitements d'extraction et de dégagements non traumatiques (insert 160, figure 3C). D'autres exemples d'instruments vibratoires sont présentés notamment dans les documents US 6 312 256 et US 4 283 175.

A chaque nouveau traitement, et même à certaines étapes d'un même traitement, le praticien doit changer d'instrument sur la pièce à main, c'est-à-dire dévisser la base de l'instrument ayant servi précédemment et revisser un autre instrument adapté pour le nouveau traitement ou l'étape suivante du traitement. Au cours d'une même journée de travail, le praticien peut avoir à répéter cette opération un grand nombre de fois. Or, si cette opération n'est pas en elle-même difficile, le praticien doit tout de même y accorder à chaque fois un temps suffisant pour s'assurer du bon montage de l'instrument. En effet, pour un fonctionnement optimal, l'instrument vibratoire doit être correctement vissé sur la pièce à main et suffisamment serré afin d'obtenir un bon couplage mécanique avec le transducteur, le praticien pouvant, à cet effet, notamment utiliser une pince dynamométrique. Il existe, par conséquent, un besoin de réduire le temps et de simplifier la manipulation lors du changement d'un instrument et, plus particulièrement, d'une partie travaillante ou outil.

Le document JP 2002/065700 divulgue une brosse montée de façon amovible sur un porte-outil solidaire d'une pièce à main ultrasonique. Cependant, dans ce document le système d'accrochage de la brosse sur le porte-outil est adapté pour permettre une bonne transmission des vibrations à une brosse, c'est-à-dire à un outil non rigide, et dont la masse est importante. En outre, dans le système décrit dans ce document, les portions de couplage et d'accrochage sont confondues. Un tel système d'acrrochage ne permet pas de transmettre convenablement les vibrations ultrasonores à des outils ou parties travaillantes rigides telles que ceux décrits ci-avant.

Le document US 4 984 985 divulgue un instrument vibratoire comprenant un outil monté de façon amovible sur un porte-outil, ledit porte-outil étant destiné à être couplé mécaniquement de façon rigide avec un dispositif générateur de vibrations. Dans ce document, l'outil comporte une portion de couplage et une partie d'attache élastiquement déformable disposée en amont de la portion de couplage. Le porte-outil comporte successivement suivant un axe longitudinal un logement recevant la partie d'attache de l'outil et un élément de couplage entourant la portion de couplage de l'outil.

### Objet et résumé de l'invention

La présente invention a pour but de proposer une solution qui permet de monter de façon amovible une partie travaillante ou outil d'un instrument vibratoire sans avoir à dévisser systématiquement l'ensemble de l'instrument de la pièce à main, et ce tout en assurant une transmission optimale des vibrations (sonores ou ultrasonores) depuis la pièce à main jusqu'à la partie travaillante de l'instrument.

Ce but est atteint grâce à un instrument vibratoire comprenant un outil monté de façon amovible sur un porte-outil, ledit porte-outil étant destiné à être couplé mécaniquement de façon rigide avec un dispositif générateur de vibrations,
l'outil comportant successivement suivant un axe longitudinal une partie travaillante présentant une extrémité libre destinée à reproduire les vibrations transmises par le dispositif de vibrations, une portion de couplage ainsi qu'une partie d'attache élastiquement déformable et disposée en amont de la portion de couplage,
le porte-outil comportant successivement suivant l'axe longitudinal un logement recevant la partie d'attache dudit outil et un palier de couplage entourant au moins partiellement la portion de couplage de l'outil,
la partie d'attache de l'outil présentant des dimensions extérieures adaptées par rapport aux dimensions intérieures du logement du porte-outil de manière à empêcher un contact entre ladite partie d'attache et ledit logement lorsque la portion de couplage de l'outil est en contact avec le palier de couplage du porte-outil.

Ainsi, lorsqu'une force d'appui axiale et/ou radiale est appliquée sur la partie travaillante de l'outil, c'est-à-dire lorsque la partie travaillante est mise en contact avec l'objet à traiter, par exemple une dent, le seul contact entre le porte-outil et l'outil se situe entre la portion de couplage de l'outil et le palier de couplage du porte-outil. On assure, par conséquent, une transmission optimale des vibrations par le porte-outil à l'outil puisqu'on empêche l'amortissement de ces dernières qui pourraient se produire lorsque des parties de l'outil et du porte-outil autres que les éléments de couplage adaptés sont en contact.

La transmission optimale des vibrations entre le porte-outil et l'outil est également assurée par le fait que la portion de couplage de l'outil est disposée entre la partie d'attache et la partie travaillante de ce dernier. Ainsi, les vibrations reçues depuis le palier de couplage du porte-outil par la portion de couplage de l'outil sont directement transmises à la partie travaillante sans avoir à cheminer à travers d'autres parties de l'outil susceptibles de diminuer l'amplitude des ondes ultrasonores.

En outre, grâce à sa partie d'attache élastiquement déformable, l'outil de la présente invention peut être facilement et rapidement assemblé au porte-outil. De même, l'outil selon la présente invention peut être retiré tout aussi rapidement et facilement du porte-outil. Par conséquent, puisque dans la présente invention la partie travaillante de l'instrument vibratoire est intégrée à un outil qui peut être rapidement et facilement monté et démonté d'un porte-outil solidaire du dispositif générateur de vibrations, le changement de partie travaillante peut être réalisé en un temps très bref, en particulier par rapport au temps requis pour le changement de la partie travaillante avec les instruments vibratoires de l'art antérieur qui impliquent le démontage et le montage complets respectivement de deux instruments différents au niveau du dispositif générateur de vibrations.

Selon des variantes de réalisation de l'instrument vibratoire selon l'invention, la portion de couplage de l'outil et le palier de couplage du porte-outil peuvent présenter des formes tronconiques complémentaires, ou des formes cylindriques, ou encore à la fois des premières portions de forme tronconique complémentaire et des deuxièmes portions de forme cylindrique.

Dans le cas où la portion de couplage de l'outil et le palier de couplage du porte-outil présentent des formes cylindriques, la portion de couplage de l'outil comportant à son extrémité jointive avec la partie travaillante dudit outil une butée axiale, ladite butée axiale présentant un diamètre supérieur au diamètre du palier de couplage du porte-outil. Dans le cas où la portion de couplage de l'outil et le palier de couplage du porte-outil présentent des formes tronconiques complémentaires, ces deux éléments forment eux-mêmes un système de butée axiale.

Selon une caractéristique supplémentaire de l'invention, la partie d'attache de l'outil comprend un élément de retenue disposé en amont de la portion de couplage et en ce que le logement du porte-outil comporte une portion de retenue recevant l'élément de retenue de l'outil.

Selon une autre caractéristique supplémentaire de l'invention, la portion de couplage de l'outil comprend une butée radiale et en ce que le porte-outil comporte un segment évidé recevant ladite butée radiale, la butée radiale et le segment évidé formant un dispositif de limitation de la rotation de l'outil dans le porte-outil.

Selon un aspect de l'invention, l'élasticité de la partie d'attache est réalisée en ménageant dans celle-ci au moins une fente longitudinale.

La partie d'attache peut comporter également au moins une portion de section réduite sensiblement au centre de ladite partie d'attache.

Selon une caractéristique additionnelle de l'invention, le porte-outil comporte un canal interne apte à coopérer avec un canal interne de ladite pièce à main et en ce que l'outil comporte un canal interne apte à coopérer avec le canal interne du porte-outil débouchant au niveau de la partie travaillante.

La présente invention a également pour objet un appareil de traitement dentaire à ultrasons comprenant au moins une pièce à main chirurgicale reliée à un générateur de vibrations, caractérisé en ce qu'il comprend en outre au moins un instrument vibratoire selon la présente invention.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels :
- la figure 1 est une vue en perspective d'un appareil de chirurgie dentaire à ultrasons,
- la figure 2 est une vue en perspective d'un instrument vibratoire avant montage sur une pièce à main selon l'art antérieur,
- les figures 3A à 3C montrent des exemples d'instruments vibratoires ayant des formes différentes,
- la figure 4 est une vue en perspective et partiellement en coupe avant montage d'un outil dans un porte-outil d'un instrument vibratoire conformément à un mode de réalisation de l'invention,
- les figures 5A à 5C illustrent une opération de montage de l'outil ultrasonique dans le porte-outil de l'instrument vibratoire de la figure 4,
- la figure 6 montre l'instrument vibratoire de la figure 5C lorsqu'une force d'appui axiale est appliquée sur la partie travaillante de l'outil,
- les figures 7 et 8 sont des vues en coupe d'un instrument vibratoire conformément à un autre mode de réalisation de l'invention,
- la figure 9 est une vue en coupe d'un instrument vibratoire conformément à encore un autre mode de réalisation de l'invention,
- les figures 10A et 10B sont respectivement une vue en perspective et une vue en coupe montrant un dispositif de limitation en rotation disposé sur l'instrument vibratoire de la figure 4,
- les figures 11A et 11B sont respectivement une vue en perspective et une vue en coupe montrant un dispositif de limitation en rotation disposé sur l'instrument vibratoire de la figure 7.

### Description détaillée des modes de réalisation de l'invention

La présente invention propose une nouvelle conception d'instrument ou insert vibratoire (encore appelé "tip" ou "sonotrode") qui est formé au moins en deux éléments séparables, à savoir un premier élément correspondant à un outil vibrant dont l'extrémité (partie travaillante) est destinée à reproduire des vibrations sonores ou ultrasonores et un porte-outil destiné à être couplé mécaniquement de façon rigide avec un dispositif générateur de vibrations sonores ou ultrasonores telle qu'une pièce à main génératrice d'ultrasons. L'outil et le porte-outil sont réalisés en des matériaux relativement rigides tels que des métaux, alliages métalliques (acier), ou des composites du type matériau carbone.

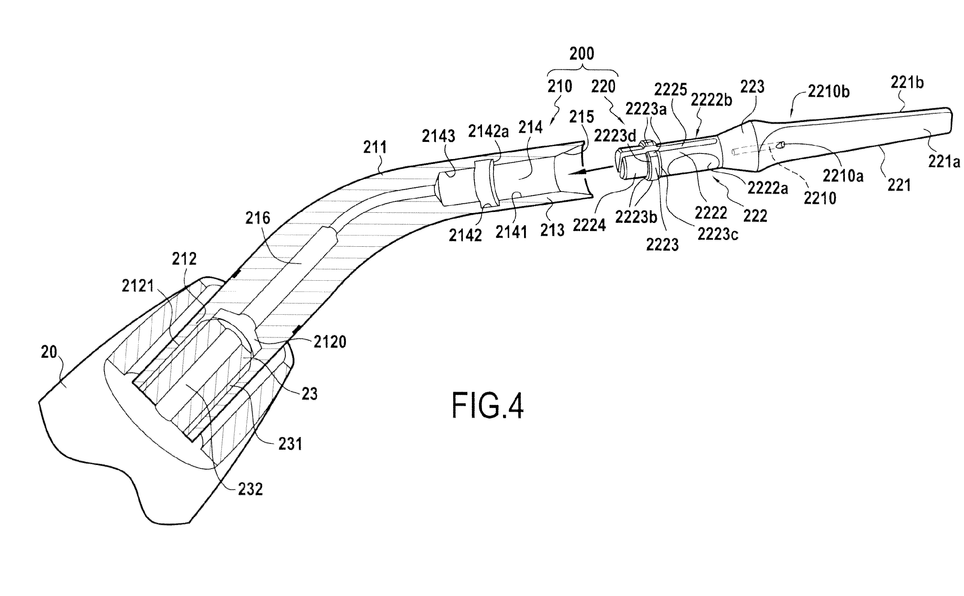

La figure 4 représente un instrument vibratoire ou insert 200 conformément à un premier mode de réalisation de la présente invention et qui comprend un porte-outil 210 et un outil 220. Le porte-outil 210 est une pièce monobloc qui comporte un corps 211 ayant une première extrémité ou base 212 destinée à être fixée de façon rigide sur une pièce à main 20. De façon bien connue, la pièce à main 20 est un générateur de vibrations sonores ou ultrasonores qui peut comprendre un transducteur (non représenté) formé par exemple d'un matériau piézoélectrique et couplé mécaniquement de façon rigide à l'insert de manière à transmettre à ce dernier des ondes vibratoires. Dans le mode de réalisation décrit ici, le porte-outil 210 est fixé de façon rigide à la pièce à main par vissage sur un élément de fixation 23 solidaire du transducteur de la pièce à main. A cet effet, la base 212 du porte-outil 210 comporte un évidement 2120 sur la paroi duquel on a réalisé un taraudage 2121 apte à coopérer avec un filetage 231 présent sur l'élément 23. Bien entendu, la forme et les moyens de fixation (ici un taraudage) de la base du porte-outil à la pièce à main peuvent être différents. D'une manière générale, la forme et les moyens de fixation de la base du porte-outil sont définis en fonction de ceux de l'élément de fixation présent sur la pièce à main. Le porte-outil peut également être fixé de façon permanente sur la pièce à main, par exemple par soudure, ou correspondre simplement à un prolongement de cette dernière. Le porte-outil présenté sur la figure 4 présente une forme courbée pour faciliter l'accès du site à traiter. Toutefois, suivant les sites concernés, le porte-outil peut présenter d'autres types de formes et notamment une forme encore plus courbée ou au contraire une forme droite.

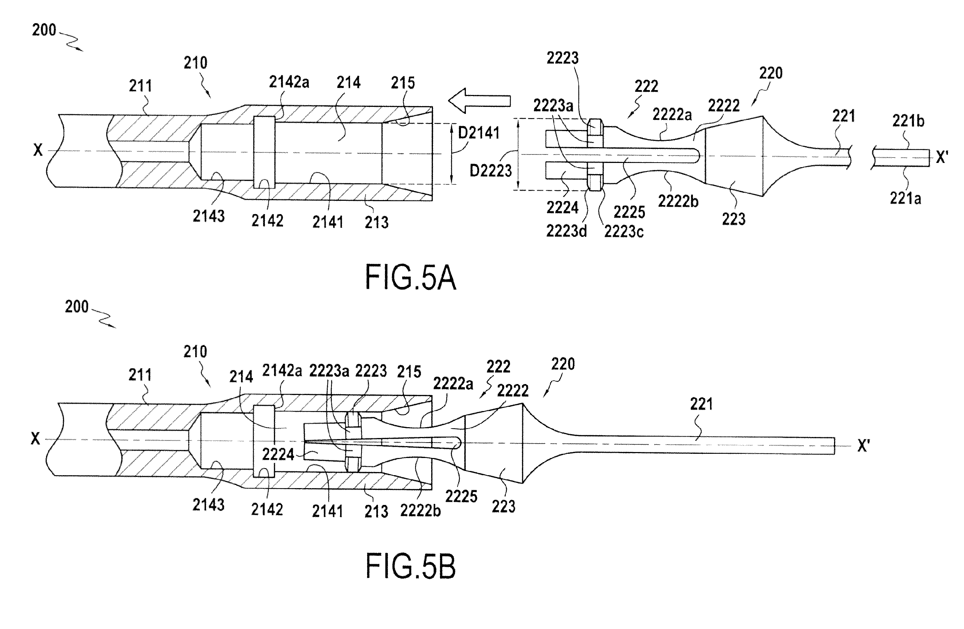

Comme illustré sur les figures 4 et 5A, Le corps 211 du porte-outil 210 s'étend depuis la base 212 jusqu'à une seconde extrémité 213 et comporte successivement suivant un axe longitudinal XX' (figure 5A) un logement 214 destiné à recevoir une partie d'attache 222 de l'outil 220 et un palier de couplage 215 destiné à entourer au moins partiellement et à coopérer avec une portion de couplage 223 de l'outil 220 comme expliqué en détail ci-après. Le logement 214 est formé par une cavité ménagée à l'intérieur du porte-outil 210 au niveau de l'extrémité 213. Le logement 214 présente une forme évolutive qui forme, depuis le palier de couplage 215 jusqu'au fond du logement 214, une portion de section réduite 2141, une portion de retenue 2142 et un fond 2143. Le palier de couplage 215, qui présente ici sur sa surface interne une forme tronconique femelle, est destiné à permettre un couplage mécanique avec l'outil 220 comme expliqué plus loin. La portion de retenue 2142 forme une gorge qui présente un rebord 2142a permettant de retenir l'outil 220 une fois celui-ci introduit dans le logement 214. Le fond 2143 est destiné à recevoir l'extrémité de la partie d'attache de l'outil 220. Le fond 2143 est relié avec le fond de l'évidement 2120 de la base 212 par un canal de circulation interne 216 ménagé à l'intérieur du corps 211 et destiné à coopérer avec un canal interne 232 de l'élément de fixation 23 de la pièce à main.

L'outil ultrasonique 220 comprend principalement trois parties disposées successivement suivant l'axe longitudinal XX', à savoir une première partie dite "partie travaillante" 221, une deuxième partie correspondant à une portion de couplage 223, et une troisième partie dite "partie d'attache" 222. La partie travaillante correspond à la partie "utile" de l'insert, c'est-à-dire à la partie avec laquelle le traitement est réalisé. La forme de la partie travaillante de l'insert et en particulier celle de son extrémité est déterminée en fonction du traitement à réaliser. Dans le mode de réalisation décrit ici, la partie travaillante 221 présente une forme aplatie définissant deux faces 221a et 221b. Cette forme convient notamment pour réaliser des débridements. La partie travaillante peut être en outre structurée et/ou comporter un revêtement de surface (par exemple un revêtement abrasif). Comme représenté sur la figure 4, l'outil 220 comporte en outre un canal interne 2210 qui débouche sur les deux faces 221a et 221b par des ouvertures 2210a et 2210b. Le canal 2210 est destiné à recevoir un fluide dispensé à partir de la pièce à main via le canal interne 232 et transmis jusqu'à l'outil par le canal interne 216 du porte-outil.

La partie d'attache 222 correspond à la partie qui est introduite dans le logement 214 du porte-outil 210. La partie d'attache 222 comporte une portion allongée 2222, un élément de retenue 2223 et une portion de guidage 2224. La partie d'attache 222 comporte en outre une fente 2225 qui s'étend longitudinalement dans la portion de guidage 2224, l'élément de retenue 2223 et la portion allongée 2222. La fente longitudinale 2225 a pour fonction de conférer à la partie d'attache 222 de l'outil 220 une capacité de déformation élastique de manière à permettre l'introduction et le retrait de l'outil 220 dans le logement 214 du porte-outil 210. De préférence, la fente 2225 s'étend uniquement dans la partie d'attache 222 et non dans la portion de couplage 223. En effet, si la fente 2225 s'étendait également dans la portion de couplage 223, elle réduirait la rigidité de la portion de couplage, ce qui pourrait avoir un effet d'amortissement sur les vibrations transitant par cette portion vers la partie travaillante de l'outil. En outre, toujours dans le cas où la fente s'étendrait jusque dans la portion de couplage, cette dernière pourrait se déformer et réduire, par conséquent, sa surface de contact (couplage) avec le palier de couplage du porte-outil.

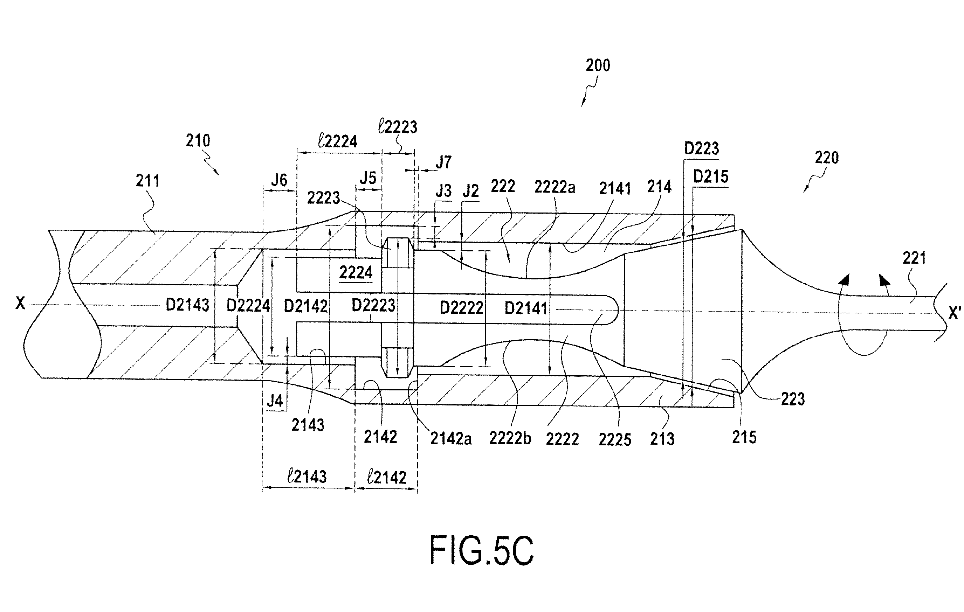

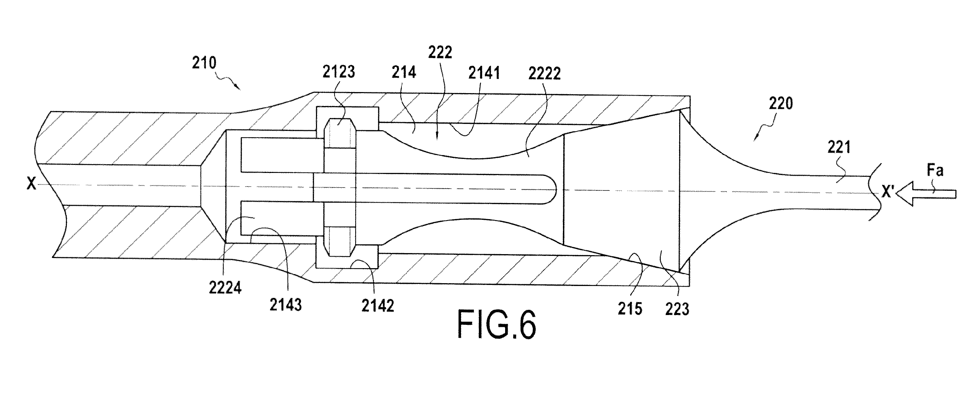

Les figures 5A à 5C montrent l'introduction de l'outil 220 dans le porte-outil 210. La partie d'attache 222 de l'outil 220 est introduite dans le logement 214 du porte-outil 210 en engageant la portion de guidage 2224 dans l'entrée du logement (figure 5A). L'élément de retenue 2223 qui forme une couronne en saillie sur la partie d'attache 222 entre alors en contact avec la paroi interne du logement qui forme au niveau de la portion de section réduite 2141 un passage ayant un diamètre D₂₁₄₁ inférieure à la largeur D₂₂₂₃ de l'élément de retenue 2223 lorsque la partie d'attache est au repos. Le passage de l'élément de retenue dans la portion de section réduite 2141 est rendu possible grâce à la présence de la fente longitudinale 2225 qui permet à la partie d'attache 222 de se déformer élastiquement ainsi qu'à la présence de méplats 2223a et 2223b ménagés sur l'élément de retenue, chacun de part et d'autre de la fente 2225 (figure 5B). Les méplats 2223a et 2223b s'étendent chacun suivant une direction perpendiculaire au plan de la fente 2225. La distance entre les méplats 2223a et 2223b est inférieure ou égale au diamètre D₂₁₄₁ de la section de portion réduite. Lorsque l'élément de retenue atteint la portion de retenue 2142, la partie d'attache 222 retrouve sa forme initiale (figure 5C et 6). L'élément de retenue 2223 se loge alors dans l'espace ménagée par la portion de retenue 2142. Le rebord 2142a empêche l'outil 220 de ressortir du logement 214 du porte-outil 210 tant qu'une force de traction significative n'est pas appliquée sur l'outil.

Conformément à la présente invention, afin d'assurer une bonne transmission des ondes ultrasonores entre le porte-outil 210 et l'outil 220, les dimensions de ces deux éléments ont été déterminées de sorte que, lorsque la portion de couplage de l'outil et le palier de couplage du porte-outil sont en contact dit "travaillant", à savoir lors d'une pression axiale et/ou radile sur la partie travaillante de l'outil, il n'y a pas d'autre contact entre le reste des autres parties de l'outil et du porte-outil, à savoir entre la partie d'attache de l'outil et le logement du porte-outil. En dehors de ce contact travaillant, l'outil est simplement retenu dans le porte-outil par l'élément de retenue de la partie d'attache.

Plus précisément, comme illustré sur la figure 5C, la portion de couplage 223 de l'outil 220 ayant une forme tronconique mâle présente un diamètre moyen D₂₂₃ tandis que la surface interne du palier de couplage 215 du porte-outil 210 ayant une forme tronconique femelle présente un diamètre moyen D₂₁₅. Ces formes complémentaires permettent un emboîtement et un couplage entre ces deux éléments. En outre, la différence entre les diamètres D₂₂₃ et D₂₁₅, et la différence de conicité entre la portion de couplage 223 et le palier de couplage 215, constituant toutes les deux la tolérance d'usinage d'emboîtement conique, influe sur la profondeur de pénétration de la portion de couplagz 223 dans le palier de couplage 215. Ces différences sont, par conséquent, déterminées de manière à ce que, lorsque la portion de couplage de l'outil et le palier de couplage du porte-outil sont en contact travaillant, aucune autre parties de ces deux éléments n'est en contact. Sur la figure 5C, la portion de couplage 223 et la surface interne du palier de couplage 215 présentent des formes tronconiques ayant sensiblement la même conicité.

L'élément de retenue 2223 de la partie d'attache 222 de l'outil présente une largeur l₂₂₂₃ inférieure à la largeur l₂₁₄₂ de la portion de retenue 2142 du logement 214 du porte-outil de manière à ménager des espaces ou jeux moyens axiaux J₅ et J₇ entre ces deux parties.

La portion de guidage 2224 de la partie d'attache 222 de l'outil présente une longueur l₂₂₂₄ inférieure à la longueur l₂₁₄₃ du fond 2143 du logement 214 de l'outil de manière à ménager un espace ou jeu moyen axial J₆ entre ces deux parties.

La portion allongée 2222 de l'outil présente, dans sa portion la plus grande, un diamètre D₂₂₂₂ inférieur au diamètre D₂₁₄₁ de la portion de section réduite 2141 du logement 214 du porte-outil de manière à ménager un espace ou jeu minimal radial J₂ entre ces deux parties.

L'élément de retenue 2223 de l'outil présente un diamètre D₂₂₂₃ inferieur au diamètre D₂₁₄₂ de la portion de retenue 2142 du logement 214 de manière à ménager un espace ou jeu moyen radial J₃ entre ces deux parties.

La portion de guidage 2224 de l'outil présente un diamètre D₂₂₂₄ inferieur au diamètre D₂₁₄₃ du fond 2143 du logement de manière à ménager un espace ou jeu moyen radial J₄ entre ces deux parties.

L'élément de retenue 2223 est disposé sur la partie d'attache 222 de l'outil à un emplacement déterminé permettant de ménager un jeu J₇ entra la face amont de l'élément de retenue et le bord amont de la portion de retenue 2142 du logement 214.

Conformément à l'invention, les jeux J₂ à J₇, présents entre les parties de la partie d'attache 222 de l'outil et les parties du logement 214 du porte-outil, sont déterminés de manière à empêcher un contact entre la partie d'attache 222 et le logement 214 lorsque la portion de couplage 223 et la surface interne du palier de couplage 215 sont en contact.

Tel que représenté sur la figure 6, lorsqu'une force axiale d'appui Fa est exercée sur la partie travaillante 221 de l'outil 220, seule la portion de couplage 223 de l'outil et le palier de couplage 215 du porte-outil 210 sont en contact travaillant, les autres parties de la partie d'attache de l'outil et du logement du porte-outil n'étant pas en contact. Ainsi, les vibrations ultrasonores reçues par le porte-outil depuis la pièce à main sont transmises à l'outil par la seule surface de contact formée entre la portion de couplage 223 et le palier de couplage 215. De cette manière, il n'y a pas de risque d'amortissement de ces vibrations par un contact entre d'autres parties de l'outil et du porte-outil, ce qui permet d'obtenir une transmission optimale des vibrations ultrasonores entre le porte-outil et l'outil. De même, lors de l'application d'une force d'appui latérale ou radiale sur la partie travaillante 221, on obtient la même configuration, à savoir seulement un contact entre la portion de couplage 223 de l'outil et le palier de couplage 215 du porte-outil 210 et aucun contact entre la partie d'attache de l'outil et le logement du porte-outil.

Le jeu J₇ entre la face amont de l'élément de retenue et le bord amont de la portion de retenue 2142 du logement 214 est inférieur à la longueur sur laquelle s'étend la portion de couplage 223 afin d'éviter à cette dernière dans certaines positions de l'instrument de sortir complètement du porte-outil et de ne plus être en regard d'au moins une partie du palier de couplage du porte-outil.

Comme expliqué précédemment, l'outil doit être réalisé en un matériau suffisamment rigide pour reproduire correctement les vibrations ultrasonores générées par la pièce à main. En conséquence, la présence de la fente longitudinale permet de donner à la partie d'attache de l'outil une capacité de déformation et une élasticité suffisantes pour permettre respectivement l'insertion et le maintien de l'outil dans le porte-outil. En effet, les deux parties de la partie d'attache séparées par la fente peuvent être rapprochées l'une de l'autre pour le passage de l'élément de retenue dans la portion de section réduite (figure 5B) et retrouver ensuite leur position initiale une fois que l'élément de retenue a atteint la portion de retenue du logement, ce qui permet d'assurer le maintien de l'outil dans le porte-outil (figure 5C).

Le retrait de l'outil du porte-outil s'opère en exerçant sur celui-ci une force de traction dirigée vers la sortie du logement, par exemple en tirant sur sa partie travaillante dans le sens opposé à celui de l'introduction. Dans ce cas, la partie d'attache 222 de l'outil 220 se déforme pour permettre à l'élément de retenue 2223 de se désengager de la portion de retenue 2142 et de passer dans la portion de section réduite 2141 comme illustré sur la figure 5B.

Afin d'accroître encore la capacité de déformation élastique de la partie d'attache 222 de l'outil 220, des portions de section réduites 2222a et 2222b (obtenues par exemple fraisage) sont réalisées au niveau de sa portion allongée 2222, ce qui permet de réduire localement l'épaisseur du matériau rigide. En outre, deux méplats 2223a sont usinés sur l'élément de retenue 2223 afin de permettre le passage de ce dernier dans le logement du porte-outil et en particulier dans la portion de section réduite 2141. Les méplats 2223a sont réalisés de part et d'autre de la fente 2225 et s'étendent suivant une direction perpendiculaire au plan de la fente.

Par ailleurs, l'élément de retenue 2223 peut comprendre des chanfreins 2223c et 2223d sur ses bords afin de faciliter l'introduction et le retrait de l'outil dans le logement du porte-outil. Le chanfrein 2223c permet en particulier de faciliter le désengagement de l'élément de retenue 2223 avec le rebord 2142a de la portion de retenue 2142 lorsqu'une traction est exercée sur l'outil.

La largeur de la fente longitudinale 2225 est déterminée en fonction de la déformation (ici un pincement) de la partie d'attache nécessaire pour permettre le passage de l'élément de retenue 2223 dans la portion de section réduite 2141.

Comme illustré sur la figure 6, le couplage mécanique entre le porte-outil 210 et l'outil 220 pour la transmission des ondes vibratoires ultrasonores est réalisé par mise en contact du palier de couplage 215 du porte-outil 210 avec la portion de couplage 223 de l'outil 220. Ces deux portions présentent dans le mode de réalisation décrit ici des formes coniques complémentaires.

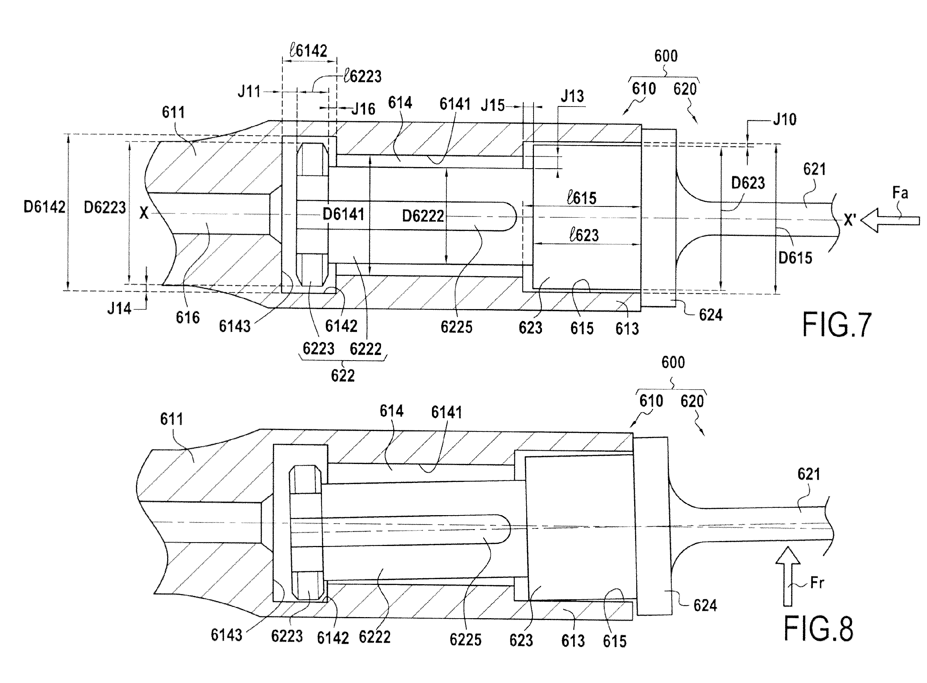

La figure 7 illustre un autre mode de réalisation d'un instrument vibratoire selon l'invention. L'instrument vibratoire de la figure 7 diffère principalement de celui présenté dans les figures 4 et 5A à 5C en ce que la portion de couplage de l'outil et le palier de couplage de l'outil présentent tous deux une forme cylindrique au lieu d'une forme tronconique comme précédemment.

De même que l'instrument vibratoire 200 décrit ci-avant, l'instrument vibratoire 600 de la figure 7, est formé d'un porte-outil 610 et d'un outil 620. Le porte-outil 610 est une pièce monobloc qui comporte un corps 611 ayant, comme le porte-outil 210 déjà décrit, une première extrémité ou base (non représentée sur la figure 7) destinée à être fixée de façon rigide sur une pièce à main. La forme et les moyens de fixation de la base du porte-outil à la pièce à main peuvent être variés (taraudage, fixation permanente, prolongement de la pièce à main, etc.)

La seconde extrémité 613 du corps 611 du porte-outil 610 comporte successivement suivant un axe longitudinal XX' un palier de couplage 615 destiné à entourer une portion de couplage 623 de l'outil 620 et à coopérer avec celle-ci et un logement 614 destiné à recevoir une partie d'attache 622 de l'outil 620. Le logement 614 présente une forme évolutive qui forme, depuis le palier de couplage 615 jusqu'au fond du logement 614, une portion de section réduite 6141, une portion de retenue 6142 et un fond 6143. Le fond 6143 est relié avec celui de la base de fixation du porte-outil (non représenté sur la figure 7) par un canal de circulation interne 616 ménagé à l'intérieur du corps 611 et destiné à coopérer avec un canal interne de l'élément de fixation de la pièce à main.

L'outil ultrasonique 620 comprend successivement suivant l'axe longitudinal XX' une partie travaillante 621, une portion de couplage 623 et une partie d'attache 622, la portion de couplage 623 comportant en outre une butée axiale 624 au niveau de sa jonction avec la partie travaillante 621. L'outil 620 peut également comporter en outre, comme l'outil 220 décrit ci-avant, un canal interne destiné à recevoir un fluide dispensé à partir de la pièce à main via le canal interne du porte-outil et qui débouche sur les faces de la partie travaillante par des ouvertures (non représentés sur la figure 7).

La partie d'attache 622 comporte une portion allongée 6222 et un élément de retenue 6223. La partie d'attache 622 comporte en outre une fente 6225 qui s'étend longitudinalement dans l'élément de retenue 6223 et la portion allongée 6222.

L'outil 620 est introduit/retiré du porte-outil 610 en exerçant une force de poussée/traction sur l'outil, la déformation élastique de la partie d'attache 622 de l'outil en raison de la présence de la fente 6225 et des méplats 2223a et 2223b permettant l'engagement/désengagement de l'élément de retenue 6223 de l'outil dans la portion de retenue 6142 du porte-outil.

En outre, conformément à l'invention, les dimensions de ces deux éléments ont été déterminées de sorte que, lorsque l'outil est monté dans le porte-outil comme représenté dans la figure 7, le jeu présent entre la portion de couplage de l'outil et le palier de couplage du porte-outil soit inférieur au jeu présent entre le reste des parties de l'outil et du porte-outil.

Plus précisément, la portion de couplage 623 de l'outil 620 présente un diamètre D₆₂₃ inférieur au diamètre D₆₁₅ du palier de couplage 615 du porte-outil 610 de manière à ménager un jeu moyen J₁₀ entre ces deux parties qui permet leur emboîtement.

L'élément de retenue 6223 de la partie d'attache 622 de l'outil présente une largeur l₆₂₂₃ inférieure à la largeur l₆₁₄₂ de la portion de retenue 6142 du logement 614 du porte-outil de manière à ménager des jeux moyens axiaux J₁₁ et J₁₆ entre ces deux parties.

La portion allongée 6222 de l'outil présente un diamètre D₆₂₂₂ inférieur au diamètre D₆₁₄₁ de la portion de section réduite 6141 du logement 614 du porte-outil de manière à ménager un jeu moyen radial J₁₃ entre ces deux parties.

L'élément de retenue 6223 de l'outil présente un diamètre D₆₂₂₃ inferieur au diamètre D₆₁₄₂ de la portion de retenue 6142 du logement 614 de manière à ménager un jeu moyen radial J₁₄ entre ces deux parties.

La portion de couplage 623 de l'outil 620 présente une longueur l₆₂₃ inférieure à la longueur l₆₁₅ du palier de couplage 615 du porte-outil 610 de manière à ménager un jeu moyen J₁₅ entre ces deux parties.

L'élément de retenue 6223 est disposé sur la partie d'attache 622 de l'outil à un emplacement déterminé permettant de ménager un jeu J₁₆ entre la face amont de l'élément de retenue et le bord amont de la portion de retenue 6142 du logement 614.

Conformément à l'invention, les jeux J₁₀, J₁₁ et J₁₃ à J₁₆, présents entre les parties de la partie d'attache 622 de l'outil et les parties du logement 614 du porte-outil, sont déterminés de manière à empêcher un contact entre la partie d'attache 622 et le logement 614 lorsque la portion de couplage 623 et la surface interne du palier de couplage 615 sont en contact travaillant.

Tel que représenté sur la figure 8, lorsqu'une force radiale d'appui Fr est exercée sur la partie travaillante 621 de l'outil 620, seuls la portion de couplage 623 de l'outil et le palier de couplage 615 du porte-outil 610 sont en contact travaillant (points de contacts ponctuels entre les deux éléments), les autres parties de la partie d'attache de l'outil et du logement du porte-outil n'étant pas en contact, en particulier grâce à la présence des jeux J₁₃, J₁₄ et J₁₆. Ainsi, les vibrations ultrasonores reçues par le porte-outil depuis la pièce à main sont transmises à l'outil par le contact entre la portion de couplage 623 et le palier de couplage 615. De cette manière, il n'y a pas de risque d'amortissement de ces vibrations par un contact entre d'autres parties de l'outil et du porte-outil, ce qui permet d'obtenir une transmission optimale des vibrations ultrasonores entre le porte-outil et l'outil.

Dans le cas de l'application d'une force axiale d'appui Fa sur la partie travaillante 621 de l'outil 620 (figure 7), le couplage mécanique entre l'outil 620 et le porte-outil 610 se fait essentiellement via la butée axiale 624 de l'outil qui vient en appui sur l'extrémité du palier de couplage 615 du porte-outil, les autres parties de la partie d'attache de l'outil et du logement du porte-outil n'étant pas en contact, en particulier grâce à la présence des jeux J₁₁ et J₁₅

Le jeu J₁₆ entre la face amont de l'élément de retenue et le bord amont de la portion de retenue 6142 du logement 614 est inférieur à la longueur sur laquelle s'étend la portion de couplage 623 afin d'éviter à cette dernière dans certaines positions de l'instrument de sortir complètement du porte-outil et de ne plus être en regard d'au moins une partie du palier de couplage du porte-outil.

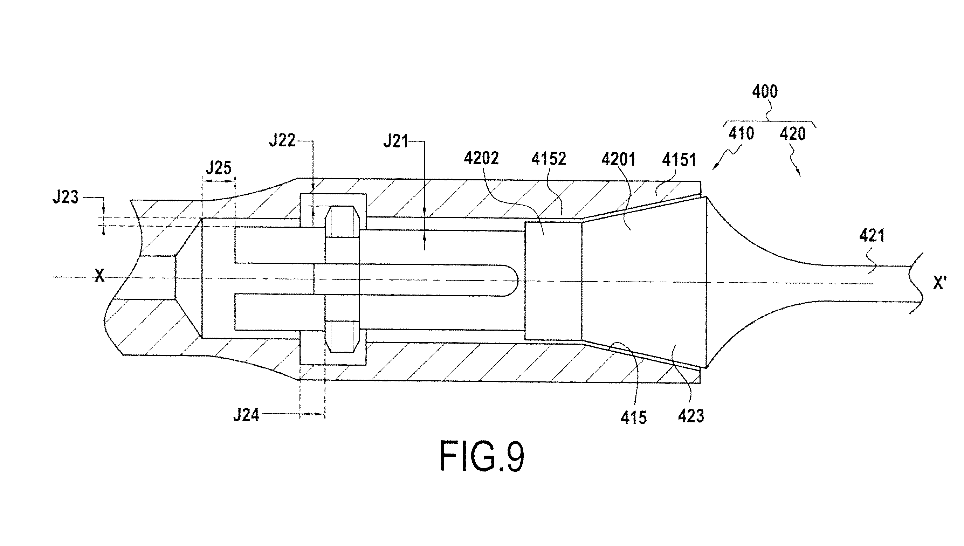

La figure 9 illustre un autre mode de réalisation d'un instrument vibratoire selon l'invention. L'instrument vibratoire 400 de la figure 9 diffère de celui présenté dans les figures 4 et 5A à 5C en ce que la portion de couplage 423 de l'outil 420 et le palier de couplage 415 de l'outil 410 présentent tous deux des formes cylindro-coniques complémentaires. Plus précisément, la portion de couplage de l'outil 420 comprend une première partie tronconique 4201 et une deuxième partie cylindrique 4202 tandis que le palier de couplage 415 du porte-outil 410 comprend également une première partie tronconique 4151 et une deuxième partie cylindrique 4152.

Les autres éléments ou parties de l'outil 420, à savoir la partie travaillante et la partie d'attache, et du porte-outil 410, à savoir le logement, sont identiques à ceux de l'instrument vibratoire 200 décrit précédemment.

Conformément à l'invention et comme expliqué précédemment, les jeux J₂₁ à J₂₅ présents entre la partie d'attache de l'outil et le logement du porte-outil ainsi que les différences de diamètre et de conicité entre la première partie tronconique 4201 de l'outil 420 et la première partie tronconique 4151 du porte-outil 410 sont définis de manière à ce que, lors de l'application d'une force d'appui axiale et/ou radiale sur la partie travaillante 421 de l'outil 420, seules la portion de couplage 423 de l'outil et le palier de couplage 415 du porte-outil soient contact travaillant, les autres parties de la partie d'attache de l'outil et du logement du porte-outil n'étant pas en contact.

Au vu des modes de réalisation décrits ci-dessus, l'homme du métier envisagera sans difficultés d'autres formes de réalisation de l'instrument vibratoire selon l'invention. D'une manière générale, afin d'optimiser la transmission des vibrations entre l'outil et le porte-outil, les dimensions de ces deux éléments sont déterminés de manière à ce que, d'une part, le ou les jeux axiaux entre la partie d'attache de l'outil et le logement du porte-outil soient supérieurs à la profondeur d'enfoncement de la portion de couplage de l'outil dans le palier de couplage du porte-outil et, d'autre part, le ou les jeux radiaux entre la partie d'attache de l'outil et le logement du porte-outil soient supérieurs à l'espace radial présent entre la portion de couplage de l'outil et le palier de couplage du porte-outil

Dans les instruments vibratoires 200, 400 et 600 décrits ci-avant, les outils 220 et 620 sont montés libre en rotation dans les porte-outils 210 et 610 en raison de la forme des surfaces des paliers de contact 2140 et 6140 desdits porte-outils et des portions de couplage 2221 et 6221 desdits outils. Dans ce cas, l'outil s'oriente automatiquement (i.e. tourne) en fonction de la surface avec laquelle il est contact. Ce mouvement de libre rotation permet, par exemple, de travailler sur toute la périphérie d'une dent avec le même outil.

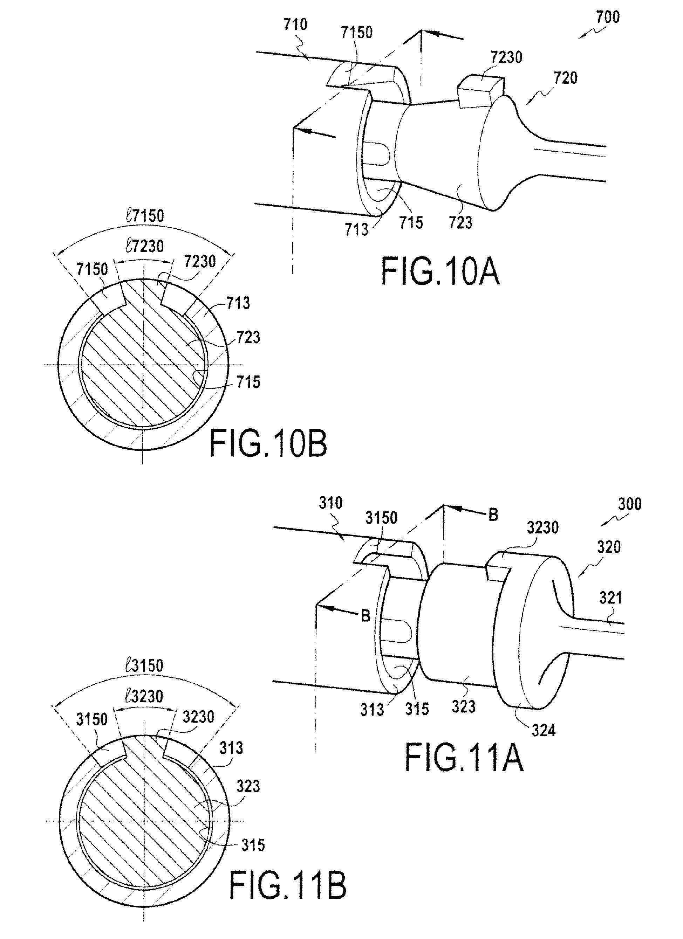

Les figures 10A et 10B illustrent un mode de réalisation d'un instrument vibratoire 700 qui diffère de l'instrument 200 présenté sur les figures 4 et 5A à 5C en ce que la portion de couplage 723 de l'outil 720 comprend une butée radiale 7230 au niveau de la partie avale de la portion de couplage et en ce que le palier de couplage 715 du porte-outil 710 présente à son extrémité libre 713 un segment évidé 7150. Lorsque l'outil 720 est introduit dans le porte-outil 710, la butée radiale 7230 se loge à l'intérieur du segment évidé pour former un dispositif de limitation de rotation de l'outil dans le porte-outil. La longueur de l'arc L₇₁₅₀ du segment évidé 7150 par rapport à la largeur l₇₂₃₀ de la butée radiale 7230 définit la limitation angulaire de rotation de l'outil 720 dans le porte-outil 710.

Les autres parties de l'outil 720 et du porte-outil 710 de l'instrument 700 sont identiques à celles de l'outil 220 et du porte-outil 210 de l'instrument 200 déjà décrit et ne seront pas décrites une nouvelle fois par souci de simplification.

Les figures 11A et 11B illustrent un mode de réalisation d'un instrument vibratoire 300 qui diffère de l'instrument 600 présenté sur la figure 8 en ce que la butée axiale 324 de la portion de couplage 323 de l'outil 320 comprend une butée radiale 3230 et en ce que le palier de couplage 315 du porte-outil 310 présente à son extrémité libre 313 un segment évidé 3150. Lorsque l'outil 320 est introduit dans le porte-outil 310, la butée radiale 3230 se loge à l'intérieur du segment évidé pour former un dispositif de limitation de rotation de l'outil dans le porte-outil. La longueur de l'arc L₃₁₅₀ du segment évidé 3150 par rapport à la largeur l₃₂₃₀ de la butée radiale 3230 définit la limitation angulaire de rotation de l'outil 320 dans le porte-outil 310.

Les autres parties de l'outil 320 et du porte-outil 310 de l'instrument 300 sont identiques à celles de l'outil 320 et du porte-outil 310 de l'instrument 300 déjà décrit et ne seront pas décrites une nouvelle fois par souci de simplification.

Dans les modes de réalisations présentés dans les figures 10A, 10B, 11A et 11B, la rotation de l'outil dans le porte-outil peut être bloquée en ayant une largeur de la butée radiale de l'outil correspondant à la longueur de l'arc du segment évidé du porte-outil.

On notera également qu'un outil ne comportant pas d'élément de butée radiale peut être également utilisé avec un porte-outil comportant un segment évidé comme décrit ci-dessus.

Au vu de la description ci-dessus, l'homme du métier envisagera sans difficultés d'autres modes de réalisation de dispositif de limitation de rotation entre l'outil et le porte-outil.

Les instruments vibratoires ou inserts de l'invention peuvent être utilisés avec des appareils périphériques à usage dentaire tels que des appareils de traitement à vibrations sonores ou ultrasonores qui, de par leur ergonomie et leurs fonctionnalités, constituent un produit fini comme celui présenté sur la figure 1 déjà décrite. Ces inserts peuvent être également mis en oeuvre avec des appareils présentés sous forme de modules destinés à être intégrés (technologie OEM) avec d'autres modules dans des produits dédiés tels que les postes de travail pour cabinet dentaire.

## Revendications

1. Instrument vibratoire (200) comprenant un outil (220) monté de façon amovible sur un porte-outil (210), ledit porte-outil étant destiné à être couplé mécaniquement de façon rigide avec un dispositif générateur de vibrations (20),
ledit outil comportant successivement suivant un axe longitudinal une partie travaillante (221) présentant une extrémité libre destinée à reproduire les vibrations transmises par le dispositif générateur de vibrations (20), une portion de couplage (223) et une partie d'attache (222) élastiquement déformable disposée en amont de la portion de couplage (223),
ledit porte-outil (210) comportant successivement suivant un axe longitudinal un logement (214) recevant la partie d'attache (222) dudit outil et un palier de couplage (215) entourant au moins partiellement la portion de couplage (223) de l'outil (220),
la partie d'attache (222) de l'outil (220) présentant des dimensions extérieures adaptées par rapport aux dimensions intérieures du logement (214) du porte-outil (210) de manière à empêcher un contact entre ladite partie d'attache et ledit logement lorsque la portion de couplage (223) de l'outil (220) est en contact avec le palier de couplage (215) du porte-outil (210).

2. Instrument vibratoire selon la revendication 1, **caractérisé en ce que** la portion de couplage (223) de l'outil (220) présente une forme tronconique et **en ce que** le palier de couplage (215) du porte-outil (210) présente une forme tronconique complémentaire de celle de ladite portion de couplage, ladite portion de couplage et le palier de couplage formant en outre un système de butée axiale.

3. Instrument vibratoire selon la revendication 1, **caractérisé en ce que** la portion de couplage (623) de l'outil (620) et le palier de couplage (615) du porte-outil (610) présentent une forme cylindrique.

4. Instrument vibratoire selon la revendication 3, caractérisé en ce la portion de couplage (623) de l'outil (620) comporte à son extrémité jointive avec la partie travaillante dudit outil une butée axiale (624) destinée à coopérer avec l'extrémité libre (613) du palier de couplage (615) du porte-outil (610).

5. Instrument vibratoire selon la revendication 1, **caractérisé en ce que** la portion de couplage (423) de l'outil (420) comporte une première portion (4201) de forme tronconique prolongée par une deuxième portion (4202) cylindrique et **en ce que** le palier de couplage (415) du porte-outil (410) comporte une première portion (4151) de forme tronconique complémentaire de celle de ladite portion de couplage et une deuxième portion (4152) de forme cylindrique.

6. Instrument vibratoire selon l'une quelconque des revendications 1 à 5, **caractérisé en ce que** la partie d'attache (222) de l'outil (220) comprend un élément de retenue (2223) disposé en amont de la portion de couplage (223) et **en ce que** le logement (214) du porte-outil (210) comporte une portion de retenue (2142) recevant l'élément de retenue (2223) de l'outil (220).

7. Instrument vibratoire selon l'une quelconque des revendications 1 à 6, **caractérisé en ce que** l'outil (320; 720) comprend une butée radiale (3230) et **en ce que** le porte-outil (310; 710) comporte un segment évidé (3150; 7150) recevant ladite butée radiale, la butée radiale et le segment évidé formant un dispositif de limitation de la rotation de l'outil (220) dans le porte-outil (210).

8. Instrument vibratoire selon l'une quelconque des revendications 1 à 7, **caractérisé en ce que** la partie d'attache (222) comporte une fente longitudinale (2225).

9. Instrument vibratoire selon la revendication 8, caractérisé en que l'élément de retenue (2223) comporte deux méplats (2223a, 2223b) chacun disposés de part et d'autre de la fente longitudinale et s'étendant suivant une direction perpendiculaire au plan de ladite fente.

10. Instrument vibratoire selon l'une quelconque des revendications 1 à 9, caractérisé en que la partie d'attache (222) comporte au moins une portion de section réduite (2222a) située sensiblement au centre de ladite partie d'attache.

11. Instrument vibratoire selon l'une quelconque des revendications 1 à 10, **caractérisé en ce que** l'outil (220) est en un matériau rigide choisi parmi au moins un métal, un alliage métallique et le carbone.

12. Instrument vibratoire selon l'une quelconque des revendications 1 à 11, **caractérisé en ce que** le porte-outil (210) comporte un canal interne (216) apte à coopérer avec un canal interne (232) de ladite pièce à main (20) et **en ce que** l'outil (220) comporte un canal interne (2210) apte à coopérer avec le canal interne (216) du porte-outil débouchant au niveau de la partie travaillante (221).

13. Appareil de traitement dentaire à ultrasons comprenant au moins une pièce à main chirurgicale reliée à un générateur de vibrations, **caractérisé en ce qu'**il comprend en outre au moins un instrument vibratoire (200) selon l'une quelconque des revendications 1 à 12.

## Patentansprüche

1. Schwingungselement (200), umfassend ein Werkzeug (220), das abnehmbar auf einem Werkzeugträger (210) montiert ist, wobei der Werkzeugträger dazu bestimmt ist, mechanisch starr mit einem Schwingungserzeuger (20) gekoppelt zu werden,
wobei das Werkzeug nacheinander entlang einer Längsachse einen arbeitenden Teil (221), der ein freies Ende aufweist, das dazu bestimmt ist, die von dem Schwingungserzeuger (20) übertragenen Schwingungen zu reproduzieren, einen Kupplungsabschnitt (223) und einen Befestigungsteil (222) umfasst, der elastisch verformbar und stromaufwärts zum Kupplungsabschnitt (223) angeordnet ist,
wobei der Werkzeugträger (210) nacheinander entlang einer Längsachse eine Aufnahme (214), die den Befestigungsteil (222) des Werkzeugs aufnimmt, und ein Kupplungslager (215) umfasst, das zumindest teilweise den Kupplungsabschnitt (223) des Werkzeugs (220) umgibt,
wobei der Befestigungsteil (222) des Werkzeugs (220) Außenabmessungen aufweist, die an die Innenabmessungen der Aufnahme (214) des Werkzeugträgers (210) angepasst sind, um einen Kontakt zwischen dem Befestigungsteil und der Aufnahme zu verhindern, wenn der Kupplungsabschnitt (223) des Werkzeugs (220) mit dem Kupplungslager (215) des Werkzeugträgers (210) in Kontakt ist.

2. Schwingungsinstrument nach Anspruch 1, **dadurch gekennzeichnet, dass** der Kupplungsabschnitt (223) des Werkzeugs (220) eine kegelstumpfartige Form aufweist, und dass das Kupplungslager (215) des Werkzeugträgers (210) eine kegelstumpfartige Form komplementär zu jener des Kupplungsabschnitts aufweist, wobei der Kupplungsabschnitt und das Kupplungslager ferner ein axiales Anschlagsystem bilden.

3. Schwindungsinstrument nach Anspruch 1, **dadurch gekennzeichnet, dass** der Kupplungsabschnitt (623) des Werkzeugs (620) und das Kupplungslager (615) des Werkzeugträgers (610) eine zylindrische Form aufweisen.

4. Schwindungsinstrument nach Anspruch 3, **dadurch gekennzeichnet, dass** der Kupplungsabschnitt (623) des Werkzeugs (620) an seinem an den arbeitenden Teil des Werkzeugs angrenzenden Ende einen Axialanschlag (624) umfasst, der dazu bestimmt ist, mit dem freien Ende (613) des Kupplungslagers (615) des Werkzeugträgers (610) zusammenzuwirken.

5. Schwingungsinstrument nach Anspruch 1, **dadurch gekennzeichnet, dass** der Kupplungsabschnitt (423) des Werkzeugs (420) einen ersten Abschnitt (4201) von kegelstumpfartiger Form, der durch einen zweiten zylindrischen Abschnitt (4202) verlängert ist, umfasst, und dass das Kupplungslager (415) des Werkzeugträgers (410) einen ersten Abschnitt (4151) von kegelstumpfartiger Form komplementär zu jener des Kupplungsabschnitts und einen zweiten Abschnitt (4152) von zylindrischer Form umfasst.

6. Schwingungsinstrument nach einem der Ansprüche 1 bis 5, **dadurch gekennzeichnet, dass** der Befestigungsteil (222) des Werkzeugs (220) ein Halteelement (2223) umfasst, das stromaufwärts zum Kupplungsabschnitt (223) angeordnet ist, und dass die Lagerung (214) des Werkzeugträgers (210) einen Halteabschnitt (2142) umfasst, der das Halteelement (2223) des Werkzeugs (220) aufnimmt.

7. Schwingungsinstrument nach einem der Ansprüche 1 bis 6, **dadurch gekennzeichnet, dass** das Werkzeug (320; 720) einen radialen Anschlag (3230) umfasst, und dass der Werkzeugträger (310; 710) ein ausgenommenes Segment (3150; 7150) umfasst, das den radialen Anschlag aufnimmt, wobei der radiale Anschlag und das ausgenommene Segment eine Vorrichtung zur Begrenzung der Drehung des Werkzeugs (220) in dem Werkzeugträger (210) bilden.

8. Schwingungsinstrument nach einem der Ansprüche 1 bis 7, **dadurch gekennzeichnet, dass** der Befestigungsteil (222) einen Längsschlitz (2225) umfasst.

9. Schwingungsinstrument nach Anspruch 8, **dadurch gekennzeichnet, dass** das Halteelement (2223) zwei Abflachungen (2223a, 2223b) umfasst, die jeweils beiderseits des Längsschlitzes angeordnet sind und sich in eine Richtung senkrecht auf die Ebene des Schlitzes erstrecken.

10. Schwingungsinstrument nach einem der Ansprüche 1 bis 9, **dadurch gekennzeichnet, dass** der Befestigungsteil (222) mindestens einen Abschnitt mit verringertem Querschnitt (2222a) umfasst, der sich im Wesentlichen in der Mitte des Befestigungsteils befindet.

11. Schwingungsinstrument nach einem der Ansprüche 1 bis 10, **dadurch gekennzeichnet, dass** das Werkzeug (220) aus einem starren Material ist, das unter mindestens einem Metall, einer Metalllegierung und Kohlenstoff ausgewählt ist.

12. Schwingungsinstrument nach einem der Ansprüche 1 bis 11, **dadurch gekennzeichnet, dass** der Werkzeugträger (210) einen inneren Kanal (216) umfasst, der geeignet ist, mit einem inneren Kanal (232) des Handgeräts (20) zusammenzuwirken, und dass das Werkzeug (220) einen inneren Kanal (2210) umfasst, der geeignet ist, mit dem inneren Kanal (216) des Werkzeugträges zusammenzuwirken, der im Bereich des arbeitenden Teils (221) mündet.

13. Gerät zur Zahnbehandlung mit Ultraschall, umfassend mindestens ein chirurgisches Handgerät, das mit einem Schwingungserzeuger verbunden ist, **dadurch gekennzeichnet, dass** es ferner mindestens ein Schwindungsinstrument (200) nach einem der Ansprüche 1 bis 12 umfasst.

## Claims

1. A vibratory instrument (200) comprising a tool (220) releasably mounted on a tool carrier (210), said tool carrier being designed to be mechanically coupled in rigid manner with a vibration generator device (20),
said tool comprising in succession along a longitudinal axis: a working portion (221) presenting a free end for reproducing the vibration transmitted by the vibration generator device (20); a coupling portion (223); and an elastically-deformable attachment portion (222) disposed upstream from the coupling portion (223);
said tool carrier (210) comprising in succession along a longitudinal axis: a housing (214) receiving the attachment portion (222) of said tool; and a coupling bearing (215) surrounding the coupling portion (223) of the tool (220), at least in part; and
the attachment portion (222) of the tool (220) presenting outside dimensions that are adapted relative to the inside dimensions of the housing (214) of the tool carrier (210) so as to prevent contact between said attachment portion and said housing when the coupling portion (223) of the tool (220) is in contact with the coupling bearing (215) of the tool carrier (210).

2. A vibratory instrument according to claim 1, **characterized in that** the coupling portion (223) of the tool (220) presents a frustoconical shape, and **in that** the coupling bearing (215) of the tool carrier (210) presents a frustoconical shape that is complementary to the shape of said coupling portion, said coupling portion and the coupling bearing also forming an axial abutment system.

3. A vibratory instrument according to claim 1, **characterized in that** the coupling portion (623) of the tool (620) and the coupling bearing (615) of the tool carrier (610) present a shape that is cylindrical.

4. A vibratory instrument according to claim 3, **characterized in that** the coupling portion (623) of the tool (620) includes an axial abutment (624) at its end joining the working portion of said tool, the axial abutment (624) serving to co-operate with the free end (613) of the coupling bearing (615) of the tool carrier (610).

5. A vibratory instrument according to claim 1, **characterized in that** the coupling portion (423) of the tool (420) includes a first portion (4201) of frustoconical shape that is extended by a second portion (4202) of cylindrical shape, and **in that** the coupling bearing (415) of the tool carrier (410) comprises a first portion (4151) of frustoconical shape complementary to the shape of said coupling portion, and a second portion (4152) of cylindrical shape.

6. A vibratory instrument according to any one of claims 1 to 5, **characterized in that** said attachment portion (222) of the tool (220) includes a retaining element (2223) disposed upstream from the coupling portion (223), and **in that** the housing (214) of the tool carrier (210) includes a retaining portion (2142) receiving the retaining element (2223) of the tool (220).

7. A vibratory instrument according to any one of claims 1 to 6, **characterized in that** the tool (320; 720) includes a radial abutment (3230), and **in that** the tool carrier (310; 710) includes an empty segment (3150; 7150) receiving said radial abutment, the radial abutment and the empty segment forming a rotation-limiter device for limiting turning of the tool (220) in the tool carrier (210).

8. A vibratory instrument according to any one of claims 1 to 7, **characterized in that** the attachment portion (222) includes a longitudinal slot (2225).

9. A vibratory instrument according to claim 8, **characterized in that** the retaining element (2223) comprises two flats (2223a, 2223b), each disposed on either side of the longitudinal slot and extending in a direction perpendicular to the plane of said slot.

10. A vibratory instrument according to any one of claims 1 to 9, **characterized in that** the attachment portion (222) includes at least one portion (2222a) of reduced section situated substantially in the center of said attachment portion.

11. A vibratory instrument according to any one of claims 1 to 10, **characterized in that** the tool (220) is made of a rigid material selected from at least a metal, a metal alloy, and carbon.

12. A vibratory instrument according to any one of claims 1 to 11, **characterized in that** the tool carrier (210) includes an internal channel (216) suitable for co-operating with an internal channel (232) in said handpiece (20), and **in that** the tool (220) includes an internal channel (2210) suitable for co-operating with the internal channel (216) of the tool carrier and opening out into the working portion (221).

13. An ultrasound dental treatment appliance comprising at least one surgical handpiece connected to a vibration generator and **characterized in that** it further comprises at least one vibratory instrument (200) according to any one of claims 1 to 12.
